Europäisches Patentamt

European Patent Office

Office européen des brevets

(11) Numéro de publication: **0 238 407 B1**

## (12) FASCICULE DE BREVET EUROPEEN

(45) Date de publication de fascicule du brevet: **05.08.92**

(51) Int. Cl.5: **A61K 39/39**, A61K 35/74, A61K 39/02, A61K 39/116, A61K 39/108, A61K 39/102, A61K 39/09

(21) Numéro de dépôt: **87400577.0**

(22) Date de dépôt: **16.03.87**

(54) **Procédés industriels de fabrication de vaccins ribosomaux et vaccins ribosomaux obtenus.**

(30) Priorité: **18.03.86 FR 8603829**

(43) Date de publication de la demande:
**23.09.87 Bulletin 87/39**

(45) Mention de la délivrance du brevet:
**05.08.92 Bulletin 92/32**

(84) Etats contractants désignés:
**AT BE CH DE ES FR GB GR IT LI LU NL SE**

(56) Documents cités:
**FR-A- 2 253 499**
**FR-A- 2 305 990**
**FR-A- 2 360 314**
**FR-A- 2 444 464**

**CHEMICAL ABSTRACTS, vol. 104, 1986, page 473, no. 184461w, Columbus, Ohio, US; G. NORMIER et al.: "NK-cell stimulating properties of a membrane proteoglycan from non-capsulated Klebsiella pneumoniae biotype a" & ACTA PATHOL. MICROBIOL. IMMUNOL. SCAND. SECT. C. 1985, 93C(6), 233-43**

(73) Titulaire: **PIERRE FABRE MEDICAMENT**
**45, Place Abel Gance**
**F-92100 Boulogne(FR)**

(72) Inventeur: **Dussourd D'Hinterland, Lucien**
**Domaine de Plombière**
**F-81100 Castres(FR)**
Inventeur: **Normier, Gérard**
**23, rue Francis Poulenc**
**F-81100 Castres(FR)**
Inventeur: **Pinel, Anne-Marie**
**22, rue des Capucins**
**F-81100 Castres(FR)**
Inventeur: **Durand, Jacques**
**19, rue des Grillons**
**F-81100 Castres(FR)**

(74) Mandataire: **Warcoin, Jacques et al**
**Cabinet Régimbeau 26, avenue Kléber**
**F-75116 Paris(FR)**

EP 0 238 407 B1

## Description

La présente invention concerne les procédés industriels de fabrication de vaccins ribosomaux et les vaccins ribosomaux qui en dérivent.

Les vaccins ribosomaux sont des vaccins dans lesquels la fraction antigénique est constituée par une fraction ribosomale, cette fraction ribosomale antigénique doit en général être adjuvée par une fraction qui peut être d'origine microbienne également, il s'agit la plupart du temps de glycoprotéines d'origine membranaire et/ou des polyssacharides ou lipopolysaccharides d'origine microbienne.

Le brevet antérieur de la demanderesse, n° 73 43957 du 10 décembre 1973, décrivait la préparation de vaccins à base de fractions ribosomales antigéniques adjuvées par une fraction membranaire de Klebsiella pneumoniae.

Les brevets antérieurs de la demanderesse n° 75 10252 du 2 avril 1975 et 76 24124 du 6 août 1976 décrivaient des vaccins dérivés des vaccins ribosomaux où l'ARN ribosomal ou bien les ribosomes eux-mêmes se trouvaient associés à des fractions polysaccharidiques ou lipopolysaccharidiques homologues et/ou hétérologues.

Le brevet antérieur de la demanderesse n° 78 35649 du 19 décembre 1978 décrivait la préparation de protéoglycanes membranaires bactériens utilisables notamment comme adjuvants dans les vaccins ribosomaux.

La présente invention, réalisée au Centre d'Immunologie et de Biologie Pierre FABRE, concerne de nouveaux procédés industriels pour la fabrication de fractions ribosomales microbiennes immunogéniques ainsi que des protéoglycanes membranaires de Klebsiella pneumoniae biotype a non capulé qui leur sont associés comme adjuvants.

Cette nouvelle technologie permet le développement d'une production à très grande échelle de ce type de vaccins, notamment pour des applications vétérinaires telles que celles citées à titre d'exemple.

Plus particulièrement, la présente invention concerne un procédé de préparation de protéoglycanes membranaires de bactéries choisies parmi : Klebsiella, Serratia et Corynebacterium, caractérisé en ce que, à partir d'un lysat clarifié de ladite souche bactérienne,

a) on traite le surnageant du lysat clarifié avec le CTAB ou de l'acide trichloroacétique, et

b) après traitement, on sépare le surnageant contenant les protéoglycanes membranaires purifiés des impuretés précipitées.

De façon générale, le lysat de bactéries est obtenu par broyage d'un concentrat bactérien à l'aide de moyens mécaniques et/ou pneumatiques, ces moyens de broyage devront, bien entendu, être adaptés au type de cellules microbiennes à désintégrer et des informations plus spécifiques seront données dans le cadre de la description des exemples.

Les lysats bactériens obtenus après broyage peuvent être clarifiés par toute technologie connue, mais seront de préférence clarifiés par centrifugation, par exemple, à 15 000 g, sur un séparateur, ceci pour éliminer les résidus de broyage et les germes non broyés.

Suivant le type de traitement effectué à l'étape a), on opère de la façon suivante :

- Lorsque l'on effectue le traitement au CTAB, on sépare le surnageant par centrifugation et on effectue une ultracentrifugation pour éliminer le CTAB lui-même, les sels et les contaminants de faible poids moléculaire, ceci en utilisant, par exemple, une ultrafiltration tangentielle avec une membrane coupant à 10 000 d ;

- dans le cas où le traitement est un traitement à l'acide trichloroacétique, le précipité d'impuretés est éliminé par centrifugation, par exemple sur séparateur, et le surnageant est ultrafiltré sur une membrane coupant à 10 000 d comme précédemment, mais après avoir de préférence ramené le pH au voisinage de 7, par exemple à 7,8, par addition d'une base telle que NaOH concentré.

Dans les deux cas, après ultrafiltration, on obtient une suspension concentrée de protéoglycanes membranaires, lesquels peuvent être stérilisés, par exemple par autoclavage à 120°C, puis lyophilisés pour être utilisés ultérieurement.

De façon préférentielle, les protéoglycanes membranaires adjuvants seront préparés à partir d'une souche de Klebsiella pneumoniae mutant, non capsulé, de biotype a, déposée à la Collection de l'Institut Pasteur de Paris sous le n° 145-I.IP.

La fraction ribosomale antigénique est préparée, de préférence, de la façon suivante :

a) On traite un lysat clarifié,filtré, de ladite souche par MgCl$_2$, par un acide organique ou par le CTAB ;

b) après traitement, on récupère le précipité qui est éventuellement lavé pour récupérer les ribosomes.

Le lysat clarifié, filtré, peut être obtenu comme cela a été décrit précédemment pour les protéoglycanes membranaires, toutefois le lysat clarifié est filtré, de préférence par ultrafiltration sur une membrane coupant à 0,22 μ, ceci pour éliminer en particulier les protéoglycanes membranaires et les différents résidus de broyage qui n'auraient pas été éliminés lors de la centrifugation.

Selon la nature du traitement de l'étape a), on opère de préférence de la façon suivante :

- Lorsque le traitement est effectué avec MgCl₂, on ajoute au lysat bactérien clarifié et filtré une solution de MgCl₂, de préférence 1 M, jusqu'à obtenir une concentration finale de 0,1 M en MgCl₂. On ajuste le pH à un pH acide, par exemple de l'ordre de 6, à l'aide de HCl dilué, et on laisse précipiter les ribosomes. Ce précipité est alors recueilli par exemple par centrifugation.
- Lorsque le traitement est effectué par un acide organique, il s'agit plus particulièrement d'un traitement à l'acide acétique, on ajoute l'acide acétique jusqu'à un pH de 4 puis on laisse le précipité de ribosomes se former et on le sépare par exemple par centrifugation.
- Lorsque le traitement est un traitement au CTAB, on ajoute au lysat bactérien clarifié et filtré une solution de CTAB, par exemple à 5 % (p/v), et on laisse le précipité se former. Ce précipité est récupéré par centrifugation et peut être lavé, par exemple à l'éthanol.

Le produit obtenu à la fin de l'étape b) constitue une fraction ribosomale brute qui peut être purifiée par lavage avec du dodécylsulfate de sodium, puis précipitation par exemple à l'alcool éthylique. Le précipité obtenu peut être soumis à une purification supplémentaire par chromatographie d'immuno-affinité sur colonne d'anticoprs monoclonaux immobilisés, spécifiques du déterminant antigénique vaccinant lié aux ribosomes. Les ribosomes hautement purifiés ainsi obtenus peuvent être dialysés pour éliminer les excédents de sels puis stérilisés avant d'être utilisés en association avec les protéoglycanes membranaires.

La nature des souches traitées pour obtenir les ribosomes dépend bien entendu du type de vaccins que l'on souhaite obtenir puisque ces ribosomes constituent la fraction antigénique des vaccins.

On donnera ci-après différentes formules de vaccins qui entrent plus particulièrement dans le cadre de la présente invention. De façon générale les souches mises en oeuvre proviennent, soit de prélèvements pathologiques, soit de collections de microorganismes, ces souches ont été réactivées par passage sur animaux de laboratoire et entretenues sur des milieux appropriés.

Parmi les vaccins qui peuvent être obtenus grâce au procédé selon la présente invention, il faut citer plus particulièrement :

## 1) **Vaccin contre les parodontopathies**

### - **Formule unitaire**

a) . Ribosomes d'Actinomyces viscosus ...............  3 µg

    . Ribosomes d'Actinomyces naeslundii .............  2 µg

    . Ribosomes de Veillonela parvula ...............  2 µg

    . Protéoglycanes membranaires de Klebsiella
      pneumoniae biotype a ........................... 15 µg

## 2) Vaccin intestinal

### - Formule unitaire

b) . Ribosomes d'Escherichia coli .................. 3 µg

. Ribosomes de Salmonella typhimurium ........... 3 µg

. Ribosomes de Shigella dysenteriae ............. 3 µg

. Ribosomes de Staphylococcus aureus ........... 3 µg

. Protéoglycanes membranaires de Klebsiella pneumoniae ..................................... 18 µg

## 3) Vaccin contre les Candidoses

### - Formule unitaire

c) . Ribosomes de Candida albicans type A ........... 3,5 µg

. Ribosomes de Candida albicans type B ........... 3,5 µg

. Protéoglycanes membranaires de Klebsiella pneumoniae ..................................... 15,0 µg

## 4) Vaccin contre la méningite

### - Formule unitaire

d) . Ribosomes de Neisseria meningitidis groupe A .... 3,5 µg

. Ribosomes de Neisseria meningitidis groupe C .... 3,5 µg

. Protéoglycanes membranaires de Klebsiella pneumoniae ..................................... 15,0 µg

### - Formule unitaire

e) . Ribosomes d'Haemophilus influenzae type a ....... 3,5 µg

. Ribosomes d'Haemophilus influenzae type b ....... 3,5 µg

. Protéoglycanes membranaires de Klebsiella pneumoniae ..................................... 15,0 µg

## 5) Vaccin pneumococcique

### - Formule unitaire

f) . Ribosomes de Streptococcus pneumoniae type 1 .... 3 µg

. Ribosomes de Streptococcus pneumoniae type 2 .... 3 µg

. Ribosomes de Streptococcus pneumoniae type 4 .... 3 µg

. Ribosomes de Streptococcus pneumoniae type 19 ... 3 µg

. Protéoglycanes membranaires de Klebsiella pneumoniae ..................................... 20 µg.

## 6) <u>Vaccin Streptococcique trivalent contre la méningite du porc</u>

### - <u>formule unitaire</u>

g) . Ribosomes de Streptococcus pyogenes groupe L ...... 5 µg

. Ribosomes de Streptococcus pyogenes groupe C ...... 5 µg

. Ribosomes de Streptococcus suis ................... 5 µg

. Protéoglycanes membranaires de Klebsiella
  pneumoniae biotype a ............................. 10 µg

## 7) <u>Vaccin contre la staphylococcie du lapin</u>

### - <u>formule unitaire vaccin trivalent</u>

h) . Ribosomes de Staphylococcus épidermidis ......... 1,5 µg

. Ribosomes de Staphylococcus aureus type 7 ....... 1,5 µg

. Ribosomes de Staphylococcus aureus ............. 1,5 µg

. Protéoglycanes membranaires de Klebsiella
  pneumoniae biotype a ........................... 7,5 µg

### - <u>formule unitaire vaccin divalent</u>

i) . Ribosomes de Staphylocoque  (souche Mérimée).... 3,5 µg

. Ribosomes de Staphylococcus aureus ............. 3,5 µg

. Protéoglycanes membranaires de Klebsiella
  pneumoniae biotype a ........................... 15,0 µg

## 8) <u>Vaccin contre la mammite de la vache</u>

### - <u>Formule unitaire</u>

j) . Ribosomes de Staphylococcus aureus ............. 5 µg

. Ribosomes de Staphylococcus uberis ............. 5 µg

. Ribosomes de Streptococcus dysgalactiae ........ 5 µg

. Protéoglycanes membranaires de Klebsiella
  pneumoniae biotype a ........................... 20 µg

Ces vaccins sont obtenus par mélange des protéoglycanes membranaires obtenus précédemment et des ribosomes obtenus par les procédés décrits, les rapports en poids entre les ribosomes et les protéoglycanes membranaires étant de préférence compris entre 1/1 et 1/3 comptés pour l'ensemble de la fraction ribosomale par rapport à la fraction protéoglycane membranaire.

Les exemples ci-après sont plus particulièrement destinés à mettre en évidence d'autres caractéristiques et avantages de la présente invention.

EXEMPLE 1 - OBTENTION DE LYSATS CLARIFIES BACTERIENS

Cet exemple décrit le procédé préféré de préparation des lysats bactériens clarifiés, que ceux-ci soient destinés à être utilisés pour la préparation de protéoglycanes membranaires ou pour la préparation de fractions ribosomales, ceci explique pourquoi il n'est pas fait mention explicitement de souches de microorganismes.

Les biomasses microbiennes sont obtenues par culture en fermenteur en milieu liquide dans des conditions classiques. La seule contrainte spécifique étant l'arrêt brusque de la croissance par refroidissement à + 4°C en fin de phase exponentielle de croissance pour préserver l'intégrité des structures biologiques.

La biomasse est séparée du milieu de culture par centrifugation continue à froid sur des séparateurs industriels de type Sharples ou Westfalia.

Après lavage par remise en suspension dans du sérum physiologique stérile et centrifugation, les concentrats cellulaires sont stockés congelés en attendant d'être traités.

Le concentrat bactérien est décongelé dans un réacteur et mis en suspension dans du tampon tris-HCl (10 mM) , pH 7,0, contenant MgCl₂ (0,15 M) à 4°C pour avoir une concentration finale de 50 g de cellules sèches par litre de suspension. On ajoute ensuite 5 mg de DNase exempte de RNase par litre de suspension.

Les cellules microbiennes sont ensuite désintégrées par passage en continu sur des broyeurs industriels de type APV Manton Gaulin à triple effet pour les souches peu résistantes ou sur homogéniseur à microbilles de verre de type DYNO MILL pour les cocci et les souches très résistantes. Cette opération est conduite à basse température ≦ 4°C au moyen d'un échangeur thermique puissant.

Les lysats bactériens obtenus précédemment sont soumis à une première clarification continue à 15 000 g sur séparateur Sharples à +4°C pour éliminer les résidus de broyage et les germes non broyés.

Le culot de centrifugation est éliminé et le surnageant recueilli, il constitue le lysat clarifié.

OBTENTION DES PROTEOGLYCANES MEMBRANAIRES DE KLEBSIELLA PNEUMONIAE (PGM-Kp) - ADJUVANTS DES RIBOSOMES

Ces procédés permettant la production à très grande échelle des (PGM-Kp) sont décrits ci-après à partir du lysat bactérien de Klebsiella pneumoniae clarifié sur Sharples obtenu à l'exemple 1.

EXEMPLE 2

50 l de surnageant Sharples (lysat bactérien clarifié) de l'exemple 1 obtenu en partant de Klebsiella pneumoniae n° 145 sont placés dans un réacteur sous agitation à 4°C. On ajoute progressivement à cette suspension 1 volume d'une solution à 1 % (p/v) de bromure de cétyltriméthylammonium (CTAB) puis on laisse au repos pendant quelques minutes.

Le précipité d'impuretés (acides nucléiques, protéines, polysaccharides acides, etc.) est éliminé par centrifugation continue sur séparateur Sharples et les surnageants contenant les (PGM-Kp) sont recueillis.

Les (PGM-Kp) sont ensuite purifiés par une étape d'ultrafiltration tangentielle sur un appareil Millipore équipé de membranes coupant à 10 000 d. Cette étape d'ultrafiltration, de dialyse et de concentration permet l'élimination quantitative du CTAB, des sels et des contaminants de faible poids moléculaire.

La suspension concentrée de (PGM-Kp) ainsi obtenue est stérilisée par autoclavage 20 minutes à 120°C puis lyophilisée stérilement.

EXEMPLE 3

50 l de surnageant Sharples (lysat bactérien clarifié de l'exemple 1 obtenu en partant de Klebsiella pneumoniae n° 145) sont placés dans un réacteur sous agitation à +4°C. On ajoute dans le réacteur 2 500 g d'acide trichloroacétique cristallisé puis on agite jusqu'à dissolution complète.

Après un repos de 20 minutes à 4°C, le précipité d'impuretés est éliminé par centrifugation continue sur séparateur Sharples et le surnageant contenant les (PGM-Kp) est recueilli.

Le pH de la solution est ensuite remonté à pH 7,8 par addition de NaOH concentrée, puis on procède à une ultrafiltration tangentielle sur membrane coupant à 10 000 d comme pour l'exemple 2.

La suspension concentrée de (PGM-Kp) ainsi obtenue est stérilisée par autoclavage 20 minutes à 120°C puis lyophilisée stérilement.

OBTENTION DE LA FRACTION RIBOSOMALE BRUTE

Ces procédés ont tous pour principe commun d'entraîner une précipitation spécifique des ribosomes permettant leur séparation par centrifugation continue à faible vitesse et évitant d'avoir recours à l'ultracentrifugation à très grande vitesse qui ne peut s'appliquer qu'à de petits volumes.

Les exemples qui suivent ont été mis en oeuvre à partir de lysats bactériens obtenus comme cela est décrit dans l'exemple 1 en partant de la souche bactérienne appropriée, le surnageant recueilli étant soumis à une ultrafiltration tangentielle sur un système Millipore équipé de membranes coupant 0,22 $\mu$.

Le filtrat limpide obtenu contient alors les ribosomes et les substances solubles qui vont être séparés comme cela sera décrit ci-après.

EXEMPLE 4

50 l de lysat bactérien ultrafiltré sont placés dans un réacteur à +4°C et additionnés lentement d'une solution 1 M de MgCl₂ jusqu'à obtenir une concentration finale de 0,1 M en MgCl₂. Le pH est alors porté à 6,0 par HCl dilué et les ribosomes sont laissés en précipitation pendant une heure à 4°C sous agitation très lente.

Le précipité est recueilli par centrifugation continue sur Sharples puis remis en solution dans un volume initial de tampon Tris-HCl (10 mM) pH 7,0 contenant EDTA Na$_2$ (5 mM).

Cette solution constitue la fraction ribosomale brute.

EXEMPLE 5

50 l de lysat bactérien ultrafiltré sont placés dans un réacteur à +4°C et le pH de la suspension est abaissé progressivement jusqu'à pH 4,0 par addition d'acide acétique (environ 0,5 % du volume).

Après 1 heure de repos à +4°C, le précipité de ribosomes bruts est séparé par centrifugation continue sur séparateur Sharples.

Le culot est dispersé énergiquement dans un volume initial de tampon Tris-HCl (10 mM) pH 7,5 contenant NaCl (0,15 M) et EDTA Na$_2$ (1 mM) en maintenant un pH constant.

Cette solution constitue la fraction ribosomale brute.

EXEMPLE 6

50 l de lysat bactérien ultrafiltré sont placés dans un réacteur à +4°C sous agitation. On ajoute lentement 5 l d'une solution à 5 % (p/v) de CTAB (bromure de cétyltriméthylammonium).

Après un repos de quelques minutes, le précipité est recueilli par centrifugation continue sur Sharples.

Le précipité est lavé deux fois par dispersion 30 minutes dans 30 l d'éthanol 70 %, 0,2 M en CH$_3$ CO ONa, pH 7,0 puis centrifugation sur Sharples.

Le culot lavé est repris dans 30 l de tampon Tris-HCl (10 mM) pH 7,0 contenant MgCl$_2$ (10 mM) et NaCl (0,15 M).

Cette solution constitue la fraction ribosomale brute.

EXEMPLE 7 - OBTENTION DES RIBOSOMES PURIFIES

La suspension ribosomale brute obtenue par l'un des trois procédés décrits précédemment est soumise à une purification comportant les étapes suivantes :

1) Lavage des ribosomes

Les impuretés liées aux ribosomes sont éliminées par lavage au SDS (Sodium Dodécyl Sulfate) dans les conditions suivantes : 30 l de solution de ribosomes bruts sont placés dans un réacteur sous agitation et la température portée à 20°C. On ajoute à cette solution 75 g de SDS puis on poursuit l'agitation pendant 45 minutes à cette température, le milieu devient rapidement limpide.

2) Précipitation des ribosomes purifiés

A la solution précédente, on ajoute rapidement sous agitation 21 l d'alcool éthylique glacé, puis on laisse au repos 30 minutes à 4°C.

Le précipité de ribosomes purifiés est recueilli par centrifugation continue sur séparateur Sharples et le culot est repris dans 10 l de tampon pH 7,0 glacé contenant MgCl$_2$ (1 mM) et KCl (0,05 M).

3) Purification par chromatographie d'affinité

Des anticorps monoclonaux sont préparés contre chaque ribosome par hyperimmunisation de la souris avec ces ribosomes et fusion des cellules spléniques immunes de la souris avec des cellules de myélome x 63 selon la technique décrite par Galfre G. et Milstein C. (Methods in Enzymol. 73 B, 3-46 (1981)). Les anticorps monoclonaux ainsi obtenus sont sélectionnés en fonction de leur affinité pour les ribosomes et de leur capacité d'induire une protection passive chez la souris contre une infection homologue.

Un support d'affinité spécifique est alors préparé par couplage covalent de cet anticorps monoclonal sur une matrice de type dextran activée par un procédé classique comme le CNBt par exemple.

Une colonne d'affinité est ainsi préparée permettant l'isolement sélectif des ribosomes immunogéniques selon le protocole décrit ci-dessous :

. Une colonne de 70 mm de diamètre sur 400 mm de hauteur est moulée avec le support d'affinité préparé comme décrit ci-dessus et équilibrée en tampon Tris-HCl 10 mM pH 7,0 contenant MgCl$_2$ (1 mM) et KCl 0,05 M.

. La solution de ribosomes lavés préparée précédemment est passée sur la colonne pour fixer les ribosomes. La colonne est ensuite lavée par le même tampon jusqu'à ce que l'effluent n'absorbe plus à 280 nm.

. Les ribosomes sont ensuite élués par le même tampon contenant en plus KSCN (2M). Le pic de ribosome détecté par son absorption à 260 nm est recueilli.

4) Ultrafiltration

La fraction ribosomale ainsi purifiée est soumise à une ultrafiltration sur membrane coupant à 100 000 d avec alimentation en tampon pH 7,0 glacé contenant MgCl$_2$ (1 mM) et KCl (0,05 M) pour éliminer totalement le KSCN et concentrer la solution pour avoir environ 10 mg/ml de ribosomes.

EXEMPLE 8 - PREPARATION DU VACCIN RIBOSOMAL

Pour la réalisation du vaccin ribosomal dans sa présentation définitive, on procède dans l'enceinte d'un bloc stérile aux opérations suivantes :
- décongélation des suspensions ribosomales,
- regroupement dans les proportions de la formule considérée en fonction de leurs titres respectifs en ribosomes,
- addition de la quantité correspondante de protéoglycanes membranaires de Klebsiella pneumoniae lyophilisés.
- homogénéisation,
- addition des excipients suivant la forme finale considérée,
- répartition en flacons unitaires (ou par multiples de la dose unitaire),
- lyophilisation stérile,
- bouchage, sertissage,
- contrôles (physico-chimiques, immunologiques, stérilité, toxicité, etc.).

**Revendications**

**1.** Procédé de préparation de vaccins ribosomaux, caractérisé en ce qu'on associe des fractions ribosomales et des protéoglycanes membranaires de bactérie dont les protéoglycanes membranaires de bactéries choisies parmi : Klebsiella, Serratia et Corynebacterium sont préparés à partir d'un lysat clarifié de ladite souche bactérienne,

a) en traitant le surnageant du lysat clarifié avec le cétyltriméthylammonium ou de l'acide trichloroacétique, et

b) après traitement en séparant le surnageant contenant les protéoglycanes membranaires purifiés des impuretés précipitées.

**2.** Procédé selon la revendication 1, caractérisé en ce que le lysat est obtenu par broyage d'un concentrat bactérien à l'aide de moyens mécaniques et/ou pneumatiques.

**3.** Procédé selon l'une des revendications 1 et 2, caractérisé en ce que le lysat est clarifié par centrifugation.

**4.** Procédé selon l'une des revendications 1 à 3, caractérisé en ce qu'après traitement au cétyltriméthylammonium, on sépare le surnageant par centrifugation et on effectue une ultrafiltration pour éliminer le cétyltriméthylammonium, les sels et les contaminants de faible poids moléculaire.

**5.** Procédé selon l'une des revendications 1 à 3, caractérisé en ce qu'après traitement à l'acide trichloroacétique, on sépare le surnageant par centrifugation et on effectue une ultrafiltration, après avoir ramené le pH entre 7 et 8, pour éliminer les sels et les contaminants de faible poids moléculaire.

**6.** Procédé selon l'une des revendications 4 et 5, caractérisé en ce que l'ultrafiltration est une ultrafiltration tangentielle sur membrane coupant à 10 000 daltons.

**7.** Procédé selon l'une des revendications 1 à 6, caractérisé en ce que la souche bactérienne est une souche de Klebsiella pneumoniae biotype a non capsulé.

**8.** Procédé de préparation selon l'une des revendications 1 à 7, caractérisé en ce que les fractions ribosomales de souches bactériennes sont préparées par un procédé caractérisé en ce que :

a) on traite un lysat clarifié, filtré, de ladite souche par $MgCl_2$, par un acide organique ou par le cétyltriméthylammonium,

b) après traitement, on récupère le précipité qui est éventuellement lavé pour récupérer les ribosomes.

**9.** Procédé selon la revendication 8, caractérisé en ce que dans l'étape a), le traitement est effectué jusqu'à une concentration de 0,05 à 0,2 M en $MgCl_2$ à un pH acide et en ce que les ribosomes précipités sont recueillis.

**10.** Procédé selon la revendication 8, caractérisé en ce que dans l'étape a), le traitement est effectué par abaissement du pH jusqu'au voisinage de 4 avec de l'acide acétique et en ce que les ribosomes précipités sont recueillis.

**11.** Procédé selon la revendication 8, caractérisé en ce que dans l'étape a), le traitement est effectué avec le cétyltriméthylammonium et en ce que dans l'étape b) le précipité est lavé par de l'éthanol.

**12.** Procédé selon l'une des revendications 8 à 11, caractérisé en ce que les ribosomes obtenus à l'étape b) sont lavés avec du dodécylsulfate de sodium puis précipités par de l'éthanol.

**13.** Procédé selon l'une des revendications 8 à 12, caractérisé en ce que le lysat clarifié est filtré par ultrafiltration sur membrane coupant à 0,22 $\mu$m.

**14.** Procédé selon l'une des revendications 1 à 13, caractérisé en ce que chaque dose unitaire de vaccins contient :

| a) | | |
|---|---|---|
| | . Ribosomes d'Actinomyces viscosus | 3 $\mu$g |
| | . Ribosomes d'Actinomyces naeslundii | 2 $\mu$g |
| | . Ribosomes de Veillonella parvula | 2 $\mu$g |
| | . Protéoglycanes membranaires de Klebsiella pneumoniae biotype a | 15 $\mu$g |

| b) | | |
|---|---|---|
| | . Ribosomes d'Escherichia coli | 3 $\mu$g |
| | . Ribosomes de Salmonella typhimurium | 3 $\mu$g |
| | . Ribosomes de Shigella dysenteriae | 3 $\mu$g |
| | . Ribosomes de Staphylococcus aureus | 3 $\mu$g |
| | . Protéoglycanes membranaires de Klebsiella pneumoniae | 18 $\mu$g |

| c) | | |
|---|---|---|
| | . Ribosomes de Candida albicans type A | 3,5 $\mu$g |
| | . Ribosomes de Candida albicans type B | 3,5 $\mu$g |
| | . Protéoglycanes membranaires de Klebsiella pneumoniae | 15,0 $\mu$g |

| d) | | |
|---|---|---|
| | . Ribosomes de Neisseria meningitidis groupe A | 3,5 $\mu$g |
| | . Ribosomes de Neisseria meningitidis groupe C | 3,5 $\mu$g |
| | . Protéoglycanes membranaires de Klebsiella pneumoniae | 15,0 $\mu$g |

| e) | . Ribosomes d'Haemophilus influenzae type a | 3,5 µg |
| | . Ribosomes d'Haemophilus influenzae type b | 3,5 µg |
| | . Protéoglycanes membranaires de Klebsiella pneumoniae | 15,0 µg |

| f) | . Ribosomes de Streptococcus pneumoniae type 1 | 3 µg |
| | . Ribosomes de Streptococcus pneumoniae type 2 | 3 µg |
| | . Ribosomes de Streptococcus pneumoniae type 4 | 3 µg |
| | . Ribosomes de Streptococcus pneumoniae type 19 | 3 µg |
| | . Protéoglycanes membranaires de Klebsiella pneumoniae | 20 µg. |

| g) | . Ribosomes de Streptococcus pyogenes groupe L | 5 µg |
| | . Ribosomes de Streptococcus pyogenes groupe C | 5 µg |
| | . Ribosomes de Streptococcus suis | 5 µg |
| | . Protéoglycanes membranaires de Klebsiella pneumoniae biotype a | 10 µg |

| h) | . Ribosomes de Staphylococcus épidermidis | 1,5 µg |
| | . Ribosomes de Staphylococcus aureus type 7 | 1,5 µg |
| | . Ribosomes de Staphylococcus aureus | 1,5 µg |
| | . Protéoglycanes membranaires de Klebsiella pneumoniae biotype a | 7,5 µg |

| i) | . Ribosomes de Staphylocoque (souche Mérimée) | 3,5 µg |
| | . Ribosomes de Staphylococcus aureus | 3,5 µg |
| | . Protéoglycanes membranaires de Klebsiella pneumoniae biotype a | 15,0 µg |

| j) | . Ribosomes de Staphylococcus aureus | 5 µg |
| | . Ribosomes de Staphylococcus uberis | 5 µg |
| | . Ribosomes de Streptococcus dysgalactiae | 5 µg |
| | . Protéoglycanes membranaires de Klebsiella pneumoniae biotype a | 20 µg |

## Claims

1. A process for preparing ribosomal vaccines characterised in that one associates ribosomal fractions and membrane proteoglycans of bacteria chosen from Klebsiella, Serratia and Corynebacterium, prepared in starting with a clarified lysate of the said bacterial strain, as follows :
   a) the supernatant of the clarified lysate is treated with cetyltrimethylammonium or with trichloroacetic acid, and
   b) after treatment, the supernatant containing the purified membrane proteoglycans is separated from the precipitated impurities.

2. The process according to claim 1, wherein the lysate is obtained by grinding a bacterial concentrate using mechanical and/or pneumatic means.

3. The process according to anyone of claims 1 and 2, wherein the lysate is clarified by centrifugation.

4. The process according to anyone of claims 1 to 3, wherein, after treatment with cetyltrimethylammonium, the supernatant is separated by centrifugation and an ultrafiltration is performed in order to remove the cetyltrimethylammonium, the salts and the low molecular weight contaminants.

5. The process according to anyone of claims 1 to 3, wherein, after treatment with trichloroacetic acid, the supernatant is separated by centrifugation and an ultrafiltration is performed, after the pH has been brought back to between 7 and 8, in order to remove the salts and the low molecular weight contaminants.

6. The process according to anyone of claims 4 and 5, wherein the ultrafiltration is a tangential ultrafiltration with a membrane which excludes at 10,000 daltons.

7. The process according to anyone of claims 1 to 6, wherein the bacterial strain is a strain of non-encapsulated Klebsiella pneumoniae biotype a.

8. A process for preparing according to anyone of claims 1 to 7 which ribosomal fractions of bacterial strains, are prepared as follows :
   a) a filtered, clarified lysate of the said strain is treated with $MgCl_2$, with an organic acid or with cetyltrimetylammonium,
   b) after treatment, the precipitate is recovered and, if appropriate, washed, in order to recover the ribosomes.

9. The process as claimed in claim 8, wherein, in stage a), the treatment is performed up to an $MgCl_2$ concentration of 0.05 to 0.2 M at an acid pH, and wherein the precipitated ribosomes are collected.

10. The process as claimed in claim 8, wherein, in stage a), the treatment is performed by lowering the pH to a value in the region of 4 with acetic acid, and wherein the precipitated ribosomes are collected.

11. The process as claimed in claim 8, wherein, in stage a), the treatment is performed with cetyltrimethylammonium, and wherein, in stage b), the precipitate is washed with ethanol.

12. The process as claimed in claim 8, wherein the ribosomes obtained in stage b) are washed with sodium dodecyl sulfate and then precipitated with ethanol.

13. The process as claimed in claim 8, wherein the clarified lysate is filtered by ultrafiltration with a membrane which excludes at 0.22 $\mu$m.

14. Process according to claims 1-13, wherein each unit dose contains :

| a) | Ribosomes of Actinomyces viscosus | 3 $\mu$g |
|---|---|---|
| | Ribosomes of Actinomyces naeslundii | 2 $\mu$g |
| | Ribosomes of Veillonella parvula | 2 $\mu$g |
| | Membrane proteoglycans of Klebsiella pneumoniae biotype a | 15 $\mu$g |

| b) | Ribosomes of Escherichia coli | 3 $\mu$g |
|---|---|---|
| | Ribosomes of Salmonella typhimurium | 3 $\mu$g |
| | Ribosomes of Shigella dysenteriae | 3 $\mu$g |
| | Ribosomes of Staphylococcus aureus | 3 $\mu$g |
| | Membrane proteoglycans of Klebsiella pneunoniae | 18 $\mu$g |

| c) | Ribosomes of Candida albicans type A | 3,5 $\mu$g |
|---|---|---|
| | Ribosomes of Candida albicans type B | 3,5 $\mu$g |
| | Membrane proteoglycans of Klebsiella pneumoniae | 15,0 $\mu$g |

| d) | Ribosomes of Neisseria meningitidis group A | 3,5 μg |
| | Ribosomes of Neisseria meningitidis group C | 3,5 μg |
| | Membrane proteoglycans of Klebsiella pneumoniae | 15,0 μg |

| e) | Ribosomes of Haemophilus influenzae type a | 3,5 μg |
| | Ribosomes of Haemophilus influenzae type b | 3,5 μg |
| | Membrane proteoglycans of Klebsiella pneumoniae | 15,0 μg |

| f) | Ribosomes of Streptococcus pneumoniae type 1 | 3 μg |
| | Ribosomes of Streptococcus pneumoniae type 2 | 3 μg |
| | Ribosomes of Streptococcus pneumoniae type 4 | 3 μg |
| | Ribosomes of Streptococcus pneumoniae type 19 | 3 μg |
| | Membrane proteoglycans of Klebsiella pneumoniae | 20 μg |

| g) | Ribosomes of Streptococcus pyogenes group L | 5 μg |
| | Ribosomes of Streptococcus pyogenes group C | 5 μg |
| | Ribosomes of Streptococcus suis | 5 μg |
| | Membrane proteoglycans of Klebsiella pneumoniae biotype a | 10 μg |

| h) | Ribosomes of Staphylococcus epidermidis | 1,5 μg |
| | Ribosomes of Staphylococcus aureus type 7 | 1,5 μg |
| | Ribosomes of Staphylococcus aureus | 1,5 μg |
| | Membrane proteoglycans of Klebsiella pneumoniae biotype a | 7,5 μg |

| i) | Ribosomes of Staphylococcus (strain Mérimée) | 3,5 μg |
| | Ribosomes of Staphylococcus aureus | 3,5 μg |
| | Membrane proteoglycans of Klebsiella pneumoniae biotype a | 15,0 μg |

| j) | Ribosomes of Staphylococcus aureus | 5 μg |
| | Ribosomes of Staphylococcus uberis | 5 μg |
| | Ribosomes of Streptococcus dysgalactiae | 5 μg |
| | Membrane proteoglycans of Klebsiella pneumoniae biotype a | 20 μg |

**Patentansprüche**

1. Verfahren zur Herstellung von ribosomalen Vakzinen, dadurch gekennzeichnet, daß man ribosomale Fraktionen und bakterielle membranäre Proteoglycane vereinigt, wobei die unter Klebsiella, Serratia und Corynebacterium ausgewählten bakteriellen membranären Proteoglycane ausgehend von einem geklärten Lysat des genannten Bakterien-Stammes hergestellt werden, und indem man

a) den aufschwimmenden Teil des geklärten Lysates mit Cetyltrimethylammonium oder Trichloressigsäure behandelt, und

b) nach der Behandlung den aufschwimmenden Teil, der die gereinigten membranären Proteoglycane enthält, von den gefällten Verunreinigungen abtrennt.

**2.** Verfahren nach Anspruch 1, dadurch gekennzeichnet, daß das Lysat durch Zerkleinerung eines Bakterien-Konzentrates mit Hilfe von mechanischen und/oder pneumatischen Mitteln erhalten wird.

**3.** Verfahren nach einem der Ansprüche 1 und 2, dadurch gekennzeichnet, daß das Lysat durch Zentrifugieren geklärt wird.

**4.** Verfahren nach einem der Ansprüche 1 bis 3, dadurch gekennzeichnet, daß man nach der Behandlung mit Cetyltrimethylammonium den aufschwimmenden Anteil mittels Zentrifugieren abtrennt und eine Ultrafiltration durchführt, um das Cetyltrimethylammonium, die Salze und die Verunreinigungen mit niedrigem Molekulargewicht zu entfernen.

**5.** Verfahren nach einem der Ansprüche 1 bis 3, dadurch gekennzeichnet, daß man nach der Behandlung mit Trichloressigsäure den aufschwimmenden Anteil mittels Zentrifugieren abtrennt und, nachdem man den pH-Wert zwischen 7 und 8 eingestellt hat, eine Ultrafiltration durchführt, um die Salze und die Verunreinigungen mit niedrigem Molekulargewicht zu entfernen.

**6.** Verfahren nach einem der Ansprüche 4 und 5, dadurch gekennzeichnet, daß die Ultrafiltration eine tangentiale Ultrafiltration mit einer Membran ist, die bei 10.000 Dalton abtrennt.

**7.** Verfahren nach einem der Ansprüche 1 bis 6, dadurch gekennzeichnet, daß der Bakterienstamm ein nicht verkapselter Stamm Klebsiella pneumonia Biotyp a ist.

**8.** Verfahren zur Herstellung nach einem der Ansprüche 1 bis 7, dadurch gekennzeichnet, daß die ribosomalen Fraktionen des Bakterienstammes durch ein Verfahren hergestellt werden, das gekennzeichnet ist durch
a) Behandlung des geklärten, filtrierten Lysates des genannten Stammes durch $MgCl_2$, durch eine organische Säure oder durch Cetyltrimethylammonium,
b) nach der Behandlung Gewinnung des Niederschlages und gegebenenfalls Waschen, um die Ribosomen zu erhalten.

**9.** Verfahren nach Anspruch 8, dadurch gekennzeichnet, daß in der Stufe a) die Behandlung bis zu einer Konzentration von 0,05 bis 0,2 M an $MgCl_2$ bei einem sauren pH-Wert durchgeführt wird und daß die ausgefällten Ribosomen gesammelt werden.

**10.** Verfahren nach Anspruch 8, dadurch gekennzeichnet, daß in der Stufe a) die Behandlung durch Absenken des pH-Wertes mittels Essigsäure bis in die Nähe von 4 durchgeführt wird und daß die ausgefällten Ribosomen gesammelt werden.

**11.** Verfahren nach Anspruch 8, dadurch gekennzeichnet, daß in der Stufe a) die Behandlung mit Cetyltrimethylammonium durchgeführt und daß in der Stufe b) der Niederschlag mit Ethanol gewaschen wird.

**12.** Verfahren nach einem der Ansprüche 8 bis 11, dadurch gekennzeichnet, daß die in Stufe b) erhaltenen Ribosomen mit Natriumdodecylsulfat gewaschen und anschließend mit Ethanol ausgefällt werden.

**13.** Verfahren nach einem der Ansprüche 8 bis 12, dadurch gekennzeichnet, daß das geklärte Lysat durch Ultrafiltration über eine Membran, die bei 0,22 $\mu$m abtrennt, filtriert wird.

**14.** Verfahren nach einem der Ansprüche 1 bis 13, dadurch gekennzeichnet, daß jede Vakzin-Einheitsdosis enthält:

| a) | . Ribosomen von Actinomyces viscosus | 3,0 $\mu$g |
|---|---|---|
| | . Ribosomen von Actinomyces naeslundii | 2,0 $\mu$g |
| | . Ribosomen von Veillonella parvula | 2,0 $\mu$g |
| | . Membranäre Proteoglycane von Klebsiella pneumoniae Biotyp a | 15,0 $\mu$g |

| b) | . Ribosomen von Escherichia coli | 3,0 $\mu$g |
|---|---|---|
| | . Ribosomen von Salmonella typhimurium | 3,0 $\mu$g |
| | . Ribosomen von Shigella dysenteriae | 3,0 $\mu$g |
| | . Ribosomen von Staphylococcus aureus | 3,0 $\mu$g |
| | . Membranäre Proteoglycane von Klebsiella pneumoniae | 18,0 $\mu$g |

| c) | . Ribosomen von Candida albicans Typ A | 3,5 $\mu$g |
|---|---|---|
| | . Ribosomen von Candida albicans Typ B | 3,5 $\mu$g |
| | . Membranäre Proteoglycane von Klebsiella pneumoniae | 15,0 $\mu$g |

| d) | . Ribosomen von Neisseria meningitidis Gruppe A | 3,5 $\mu$g |
|---|---|---|
| | . Ribosomen von Neisseria meningitidis Gruppe C | 3,5 $\mu$g |
| | . Membranäre Proteoglycane von Klebsiella pneumoniae | 15,0 $\mu$g |

| e) | . Ribosomen von Haemophilus influenzae Typ a | 3,5 $\mu$g |
|---|---|---|
| | . Ribosomen von Haemophilus influenzae Typ b | 3,5 $\mu$g |
| | . Membranäre Proteoglycane von Klebsiella pneumoniae | 15,0 $\mu$g |

| f) | . Ribosomen von Streptococcus pneumoniae Typ 1 | 3,0 $\mu$g |
|---|---|---|
| | . Ribosomen von Streptococcus pneumoniae Typ 2 | 3,0 $\mu$g |
| | . Ribosomen von Streptococcus pneumoniae Typ 4 | 3,0 $\mu$g |
| | . Ribosomen von Streptococcus pneumoniae Typ 19 | 3,0 $\mu$g |
| | . Membranäre Proteoglycane von Klebsiella pneumoniae | 20,0 $\mu$g |

| g) | . Ribosomen von Streptococcus pyogenes Gruppe L | 5,0 $\mu$g |
|---|---|---|
| | . Ribosomen von Streptococcus pyogenes Gruppe C | 5,0 $\mu$g |
| | . Ribosomen von Streptococcus suis | 5,0 $\mu$g |
| | . Membranäre Proteoglycane von Klebsiella pneumoniae Biotyp a | 10,0 $\mu$g |

| h) | . Ribosomen von Staphylococcus epidermidis | 1,5 $\mu$g |
|---|---|---|
| | . Ribosomen von Staphylococcus aureus Typ 7 | 1,5 $\mu$g |
| | . Ribosomen von Staphylococcus aureus | 1,5 $\mu$g |
| | . Membranäre Proteoglycane von Klebsiella pneumoniae Biotyp a | 7,5 $\mu$g |

| i) | . Ribosomen von Staphylokokken (Stamm Mérimée) | 3,5 $\mu$g |
|---|---|---|
| | . Ribosomen von Staphylococcus aureus | 3,5 $\mu$g |
| | . Membranäre Proteoglycane von Klebsiella pneumoniae Biotyp a | 15,0 $\mu$g |

| j) | . Ribosomen von Staphylococcus aureus | 5,0 $\mu$g |
|---|---|---|
| | . Ribosomen von Staphylococcus uberis | 5,0 $\mu$g |
| | . Ribosomen von Streptococcus dysgalactiae | 5,0 $\mu$g |
| | . Membranäre Proteoglycane von Klebsiella pneumoniae Biotyp a | 20,0 $\mu$g |